(19) Europäisches Patentamt
European Patent Office
Office européen des brevets

(11) Veröffentlichungsnummer : **0 158 875**
**B1**

(12) **EUROPÄISCHE PATENTSCHRIFT**

(45) Veröffentlichungstag der Patentschrift : **13.12.89**

(51) Int. Cl.$^4$ : **C 07 F 15/00**, C 07 F 9/50, C 07 F 9/65, B 01 J 31/24

(21) Anmeldenummer : **85103553.5**

(22) Anmeldetag : **26.03.85**

(54) **Chirale Rhodium-diphosphinkomplexe für asymmetrische Hydrierungen.**

(30) Priorität : **19.04.84 CH 1967/84**
**07.02.85 CH 547/85**

(43) Veröffentlichungstag der Anmeldung : **23.10.85 Patentblatt 85/43**

(45) Bekanntmachung des Hinweises auf die Patenterteilung : **13.12.89 Patentblatt 89/50**

(84) Benannte Vertragsstaaten : **AT BE CH DE FR GB IT LI NL**

(56) Entgegenhaltungen :
**DE-B- 2 136 470**
**GB-A- 2 114 134**
**US-A- 4 415 500**
**JOURNAL OF ORGANOMETALLIC CHEMISTRY, Band 218, 1981, Seiten 249-260, Elsevier Sequoia S.A., Lausanne, CH; K. ACHIWA "The mechanism of asymmetric hydrogenation catalysed by rhodium complexes of chiral pyrrolidinobiphosphines"**
**J.Chem.Soc.(A), (19719, 3224-3230**
**J.Org. Chem.Vol. 43, No.18, (1978), 3444-3446**
**J.of Mulecular Catalysis, 22, (1984), 283-287**
**Die Akte enthält technische Angaben, die nach dem Eingang der Anmeldung eingereicht wurden und die nicht in dieser Patentschrift enthalten sind.**

(73) Patentinhaber : **F. HOFFMANN-LA ROCHE AG**
**Postfach 3255**
**CH-4002 Basel (CH)**

(72) Erfinder : **Broger, Emil Albin, Dr.**
**Obere Egg 11**
**CH-4312 Magden (CH)**
Erfinder : **Crameri, Yvo, Dr.**
**Hohestrasse 103**
**CH-4104 Oberwil (CH)**

(74) Vertreter : **Cottong, Norbert A. et al**
**Grenzacherstrasse 124 Postfach 3255**
**CH-4002 Basel (CH)**

**EP 0 158 875 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue chirale Rhodium-diphosphinkomplexe der allgemeinen Formel

$$[Rh(X)\ (Y)\ (L_{0,1,\ oder\ 2})]_{1\ oder\ 2} \qquad (I)$$

worin X, welches gegebenenfalls auf einem Träger fixiert sein kann, einen Rest der Formel Z—COO⁻ darstellt, worin Z wiederum die Gruppe

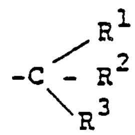

Perfluorphenyl, Perfluorbiphenyl oder einen Rest der Formel

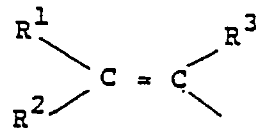

bedeutet und $R^1$, $R^2$ und $R^3$ Halogen, niederes Alkyl, Perfluorphenyl, Perfluor-$C_{1-20}$-Alkyl, Wasserstoff oder die Gruppe —COA oder AOC—$(CF_2)_n$-darstellen, worin A die Reste —OR oder —$NR'_2$ bedeutet, wobei jedoch wenigstens einer der Substituenten $R^1$, $R^2$ und $R^3$ Fluor bedeutet, R Wasserstoff, niederes Alkyl oder ein Kation, R' Wasserstoff oder niederes Alkyl, und n eine Zahl 1 bis 20 bedeuten und worin weiter Y einen chiralen Diphosphinliganden und L einen neutralen Liganden darstellen, wobei die niederen Alkylreste 1-9 Kohlenstoffatome aufweisen. Die Erfindung betrifft ferner die Herstellung der Rhodiumdiphosphinkomplexe der Formel I, sowie deren Verwendung für asymmetrische Hydrierungen.

Aus der Literatur sind bereits chirale Rhodium-diphosphinkomplexe und deren Verwendung bei asymmetrischen Hydrierungen bekannt. Ueblicherweise sind diese Komplexe kationisch oder enthalten — falls neutral — als Ligand X Chlor, Brom oder Jod. Die optischen Ausbeuten welche bei der Verwendung derartiger Komplexe in asymmetrischen Hydrierungen erzielt werden, liegen in den günstigsten Fällen bei etwa 80-84 %, im Falle der Hydrierung von Ketopantolacton [J. Org. Chem., Vol. 43, No. 18 (1978) 3444-3446].

Ueberraschenderweise wurde nun gefunden, dass die erfindungsgemässen Rhodium-diphosphinkomplexe der Formel I im Vergleich zu den vorbekannten erheblich aktiver und enantioselektiver sind, was insbesondere dazu führt, dass durch ihre Verwendung erheblich geringere Katalysatormengen verwendet werden können, kürzere Reaktionszeiten möglich sind und optische Ausbeuten von über 94 % erzielt werden können.

Der Ausdruck « niederes Alkyl » bedeutet im Rahmen der vorliegenden Erfindung geradkettige oder verzweigte Alkylgruppen mit 1 bis 9 Kohlenstoffatomen wie z. B. Methyl, Aethyl, Propyl, Isopropyl, n-Butyl, Isobutyl, tert. Butyl, Pentyl, Hexyl, Heptyl, Octyl oder Nonyl und dergleichen. Der Ausdruck « Halogen » bedeutet Fluor, Chlor, Brom und Jod, wobei Fluor bevorzugt ist. Der Ausdruck « Perfluor-$C_{1-20}$-Alkyl » bedeutet im Rahmen der vorliegenden Erfindung sowohl geradkettige, als auch verzweigte, gegebenenfalls auch optisch aktive Ketten, wobei nicht unbedingt sämtliche Wasserstoffatome durch Fluoratome ersetzt sein müssen. Falls nicht alle Wasserstoffatome durch Fluoratome ersetzt sind, so ist häufig insbesondere noch ein endständiges Wasserstoffatom vorhanden. Sofern X auf einem Träger fixiert ist, erfolgt dies über eine Gruppe —COA. Der im nachfolgenden verwendete Ausdruck « Aryl » bedeutet im Rahmen der vorliegenden Erfindung Phenyl, welches gegebenenfalls in para- und/oder meta-Stellung niedere Alkyl- oder niedere Alkoxygruppen, vorzugsweise Methyl- oder Methoxygruppen, oder auch di-niederes Alkylamino, vorzugsweise Dimethylaminogruppen, sowie eine Carboxy-, Carbamoyl-, Cyano- oder eine niedere Alkoxycarbonylgruppe aufweisen kann. Zudem können zwei Arylgruppen am gleichen Phosphoratom über die o-Stellung direkt miteinander verbunden sein oder auch über eine Methylen-, Aethylen- oder Propylengruppe. Der Ausdruck « Aryloxy » bedeutet Gruppen, in denen der Arylrest die vorhergehende Bedeutung hat. Der Ausdruck « niederes Alkoxy » bedeutet Gruppen, in denen der Alkylrest die vorhergehende Bedeutung hat. Weiterhin bedeutet das Zeichen « ◀ », dass sich der entsprechende Rest oberhalb der Molekülebene befindet, während das Zeichen « '''''' » bedeutet, dass sich der entsprechende Rest unterhalb der Molekülebene befindet. Der Buchstabe n bedeutet Zahl von 1 bis 20, vorzugsweise von 1 bis 12, insbesondere von 1 bis 8.

Der Ausdruck « neutraler Ligand » bedeutet im Rahmen der vorliegenden Erfindung einen leicht austauschbaren Liganden wie Olefine, z. B. Aethylen, Propylen, Cycloocten, 1,5-Hexadien, Norbornadien,

1,5-Cyclooctadien und dergleichen, Nitrile wie Acetonitril, Benzonitril, oder auch das verwendete Lösungsmittel usw. Dieser Ligand kann bei der Hydrierung ausgetauscht werden. Falls mehr als ein solcher Ligand vorhanden ist, können diese auch voneinander verschieden sein.

Als chirale Diphosphinliganden können im Prinzip beliebige, im Zusammenhang mit asymmetrischen Hydrierungen bekannte Diphosphinligangen, welche gegebenenfalls auch auf einem Träger fixiert sein können, verwendet werden. Derartige Liganden sind bekannt und dem Fachmann leicht zugänglich. Beispielsweise sind im Rahmen der vorliegenden Erfindung in Frage kommende Liganden bekannt aus: Markó, L. et al., Aspects of Homogeneous Catalysis, 4, 145-202 (1981); Japanische Patentanmeldung No. 67411 vom 4.6.1978 (Derwent 8180 C); deutsche Offenlegungsschrift No. 2 161 200; Europäische Patentpublikation No. 104 375. besonders geeignete und bevorzugte Liganden sind z. B. die chiralen Phosphine der allgemeinen Formel

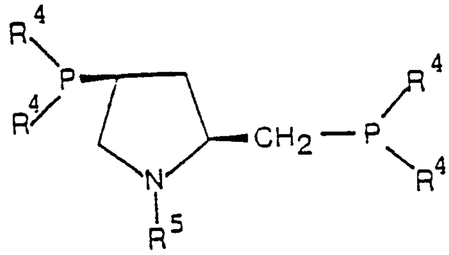

(II)

worin $R^4$ Aryl und $R^5$ die Gruppen $—CO—R^6$, $—SO_2—R^6$, $—PO(R^6)_2$ oder $—PS(R^6)_2$ darstellen, wobei $R^6$ Aryl, niederes Alkyl, di-Arylamino, di-niederes Alkylamino, Aryloxy oder niederes Alkoxy bedeuten, wobei die niederen Alkyl- bzw. Alkoxyreste 1-9 Kohlenstoffatome aufweisen und der Ausdruck Aryl für gegebenenfalls substituiertes Phenyl steht.

Bevorzugte Rhodium-diphosphinkomplexe der Formel I sind solche, worin Z die Gruppe

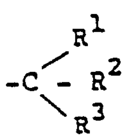

darstellt und zwei der Substituenten $R^1$, $R^2$ und $R^3$ Fluor und der andere Halogen oder Perfluor-$C_{1-20}$-Alkyl bedeuten. Bevorzugte Diphosphinliganden der Formel II sind solche, worin $R^4$ Phenyl, p-Tolyl, m-Tolyl oder 3,5-Xylyl darstellt und $R^6$ in den Resten $R^5$ Phenyl, p-Tolyl, m-Tolyl, p-nied. Alkoxycarbonylphenyl oder tert. Butoxy bedeutet. Besonders bevorzugte Phosphine sind zudem diejenigen, worin der Rest $R^5$ die Gruppe $—PO(R^6)_2$ darstellt.

Als Beispiele von bevorzugten Diphosphinliganden können folgende genannt werden:

(2S,4S)-4-(Di-m-Tolylphosphino)-2-[(di-m-tolylphosphino)methyl]-1-(diphenylphosphinoyl)-pyrrolidin; ($mCH_3$-POPPM)

(2S,4S)-4-(Diphenylphosphino)-2-[(diphenylphosphino)methyl]-1-(diphenylphosphinoyl)-pyrrolidin; (POPPM)

(2S,4S)-4-(Di-p-Tolylphosphino)-2-[(di-p-tolylphosphino)methyl]-1-(diphenylphosphinoyl)-pyrrolidin; ($pCH_3$-POPPM)

(2S,4S)-4-(Diphenylphosphino)-2-[(diphenylphosphino)methyl]-1-(di-p-carbomethoxyphenylphosphinoyl)-pyrrolidin;

(2S,4S)-4-(Diphenylphosphino)-2-[(diphenylphosphino)methyl]-1-(tert. butoxycarbonyl)-pyrrolidin; (BPPM)

(2S,4S)-4-(Di-m-Tolylphosphino)-2-[(di-m-tolylphosphino)methyl]-1-(tert. butoxycarbonyl)-pyrrolidin; ($mCH_3$-BPPM)

Besonders bevorzugte Rhodium-diphosphinkomplexe der Formel I sind diejenigen, worin $R^1$, $R^2$ oder $R^3$ Perfluor-$C_{1-20}$-Alkyl darstellen sowie diejenigen, worin Z die Gruppe $—CF_3$ oder $Cl—CF_2—$ darstellt und der Diphosphinligand BPPM; $mCH_3$-BPPM, POPPM oder $mCH_3$-POPPM ist. Ganz besonders bevorzugt sind die Komplexe der Formel

$$[Rh(CF_3\text{-}COO)\ (mCH_3\text{-}POPPM)\ (L_{0,1\ oder\ 2})]_{1\ oder\ 2}$$

$$[Rh(C_2F_5\text{-}COO)\ (mCH_3\text{-}POPPM)\ (L_{0,1\ oder\ 2})]_{1\ oder\ 2}$$

$$[Rh(C_3F_7\text{-}COO)\ (mCH_3\text{-}POPPM)\ (L_{0,1\ oder\ 2})]_{1\ oder\ 2}$$

Die erfindungsgmässen Rhodium-diphosphinkomplexe der Formel I können in an sich bekannter Weise hergestellt werden. Sie können beispielsweise dadurch hergestellt werden, dass man

a) einen Rhodiumcomplex der Formel

$$[Rh\ (X)\ (L_m)]_{1\ \text{oder}\ 2} \qquad (III)$$

worin X und L obige Bedeutung haben, und m eine Zahl von 1 bis 4 bedeutet, mit einem chiralen Diphosphinliganden umsetzt, oder

b) einen Rhodiumkomplex der Formel

$$[Rh\ (L_{m+1})]^+\ A^- \qquad (IV)$$

worin L und m obige Bedeutung haben und $A^-$ ein Anion, insbesondere $BF_4^-$, $ClO_4^-$, $PF_6^-$ oder $B(C_6H_5)_4^-$ darstellt, mit einem chiralen Diphosphinliganden und einem das Anion X enthaltenden Salz umsetzt, oder

c) einen Rhodium-diphosphinkomplex der Formel

$$[Rh\ (L_p)\ (Y)]^+\ A^- \qquad (V)$$

worin L, Y und $A^-$ obige Bedeutung haben und p eine Zahl von 1 bis 3 darstellt, mit einem das Anion X enthaltenden Salz umsetzt, oder

d) einen chiralen Rhodium-diphosphinkomplex der Formel

$$[Rh\ (X^1)\ (L_{1\ \text{oder}\ 2})\ (Y)] \qquad (VI)$$

worin $X^1$ Halogen bedeutet und L und Y obige Bedeutung haben, mit einem Silbersalz oder Thalliumsalz der Formel

$$Ag{-}X \text{ oder } Tl{-}X \qquad (VII)$$

worin X obige Bedeutung hat, umsetzt.

Die Umsetzungen der Rhodiumkomplexe der Formeln III, IV, V und VI gemäss den Reaktionsvarianten a)-d) können in an sich bekannter Weise durchgeführt werden. Zweckmässig erfolgt dies in einem inerten organischen Lösungsmittel. Als Beispiele hierfür können genannt werden: aromatische Kohlenwasserstoffe wie Benzol, Toluol usw., Ester wie Essigester, usw., cyclische Aether wie Tetrahydrofuran oder Dioxan, niedere Alkohole wie Methanol, Aethanol und dergleichen oder auch Gemische hiervon. Die Umsetzung kann bei Temperaturen zwischen etwa 0 °C bis etwa 100 °C, vorzugsweise bei etwa 15 °C bis etwa 60 °C erfolgen, jedoch unter striktem Ausschluss von Sauerstoff.

Der Ausdruck « das Anion X enthaltende Salz » bedeutet im Rahmen der vorliegenden Erfindung insbesondere Ammoniumsalze, Alkalimetallsalze, Erdalkalimetallsalze sowie andere geeignete Metallsalze. Derartige Salze sind bekannte Substanzen. Um die Löslichkeit derartiger Salze in gewissen Lösungsmitteln zu erhöhen kann gegebenenfalls ein geeigneter Kronenäther zugesetzt werden.

Bei den Rhodium-diphosphinkomplexen der Formel I handelt es sich um Katalysatoren bzw. um Vorstufen hiervon. Da deren genaue chemische Struktur nicht mit Sicherheit angegeben werden kann, sind sie auch dadurch gekennzeichnet, dass sie erhältlich sind, durch Umsetzung eines Rhodiumkomplexes der Formeln III-VI gemäss den vorhergehend erwähnten Reaktionen a) bis d).

Die als Ausgangsmaterialien verwendeten Rhodiumkomplexe der Formeln III, IV, V und VI sind bekannte Substanzen oder Analoge bekannter Substanzen, welche leicht in Analogie zu den bekannten hergestellt werden können.

Wie bereits erwähnt dienen die erfindungsgemässen Rhodium-diphosphinkomplexe der Formel I als Katalysatoren bei asymmetrischen Hydrierungen. Besonders interessant sind sie im Zusammenhang mit der asymmetrischen Hydrierung von α,β-ungesättigten Säuren und Estern sowie von α-Keto-carbonsäuren und Estern und von α-Keto-Lactonen. Insbesondere sind sie von Interesse zur asymmetrischen Hydrierung von Dihydro-4,4-dimethyl-2,3-furandion (Ketopantolacton) zum entsprechenden R-(α-Hydroxy-β,β-dimethyl-γ-butyrolacton) [R-(—)-Pantolacton].

Zur Durchführung der erwähnten asymmetrischen Hydrierungen können die Komplexe der Formel I als solche zu einer Lösung einer asymmetrisch zu hydrierenden Verbindung gegeben werden. Andererseits können sie auch in situ in Gegenwart einer asymmetrisch zu hydrierenden Verbindung gebildet werden.

Die asymmetrischen Hydrierungen können in geeigneten, unter den Reaktionsbedingungen inerten organischen Lösungsmitteln durchgeführt werden. Als solche können insbesondere genannt werden niedere Alkanole wie z. B. Methanol oder Aethanol, aromatische Kohlenwasserstoffe wie Benzol oder Toluol, cyclische Aether wie Tetrahydrofuran oder Dioxan, Ester wie z. B. Essigester oder auch Gemische hiervon und dergleichen. Das Verhältnis zwischen Rhodium und den Liganden Y liegt zweckmässig

zwischen etwa 0,05 und etwa 5 Mol, vorzugsweise zwischen etwa 0,5 und etwa 2 Mol Rhodium pro Mol Ligand. Das Verhältnis zwischen Rhodium und dem Rest X liegt zweckmässig zwischen etwa 0,01 und etwa 20, vorzugsweise zwischen etwa 0,5 und etwa 10 Mol Rhodium pro Mol Rest X. Das Verhältnis zwischen Rhodium, in den Komplexen der Formel I, und den zu hydrierenden Verbindungen liegt zweckmässig zwischen etwa 0,00001 und etwa 5 Gew.%, vorzugsweise zwischen etwa 0,0001 und etwa 0,01 Gew.%.

Die asymmetrischen Hydrierungen unter Verwendung der Komplexe der Formel I können zweckmässig bei Temperaturen von etwa 0 °C bis etwa 100 °C, vorzugsweise von etwa 20 °C bis etwa 70 °C, durchgeführt werden. Diese Hydrierungen erfolgen zweckmässig unter Druck, insbesondere unter einem Druck von etwa 1 bis 100 bar, vorzugsweise 2 bis 50 bar.

Die folgenden Beispiele dienen zur Erläuterung der Erfindung und stellen keinerlei Einschränkung hiervon dar.

In diesen Beispielen haben die verwendeten Abkürzungen folgende Bedeutung :

COD = 1,5-Cyclooctadien

BPPM ; $mCH_3$-BPPM ; POPPM ; $mCH_3$-POPPM : auf Seite 5 erwähnte Diphosphine.

Die optischen Drehungen von (R)-(—)-Pantolacton bei 589 nm (D-Linie) wurden bei 20 °C und einer Konzentration von 3 % in ionenfreiem Wasser gemessen.

Die Werte für die optischen Reinheiten basieren auf $[\alpha]_D^{20}$ = — 51,6° (c = 3, $H_2O$) für reinstes (R)-(—)-Pantolacton.

Beispiel 1

In einer Glove-Box ($O_2$-Gehalt < 1 ppm) wurden 90,5 mg (0,125 mMol) 98 %iges (2S,4S)-4-(Di-m-Tolylphosphino)-2-[(di-m-tolylphosphino)methyl]-1-(diphenylphosphinoyl)-pyrrolidin (= $mCH_3$-POPPM), 50,2 mg (0,124 mMol) Bis(1,5-Cyclooctadien)rhodium(I)tetrafluoroborat und 44,4 mg (0,125 mMol) Tetrabutylammoniumtrifluoracetat in einen 100 ml Messkolben gegeben. Dann wurde mit ca. 100 ml $O_2$-freiem Toluol auf die 100 ml-Marke aufgefüllt. Die erhaltene Suspension wurde anschliessend während 24 Stunden bei 22 °C gerührt, wobei sich eine orange trübe Lösung bildete.

Beispiel 2

200 g (1,561 Mol) Dihydro-4,4-dimethyl-2,3-furandion (Ketopantolacton) und 600 ml Toluol wurden in einem 1-Liter-Rührautoklaven vorgelegt. Nach dem Aufheizen auf 40 °C, wurde zur Entfernung der Luft aus dem Autoklaven 5 mal mit einer Hochvakuumpumpe (0,05-0,1 bar) evakuiert und jeweils mit 10 bar Wasserstoff begast. Dann wurden die Temperatur der Ketopantolactonlösung auf 35 °C eingestellt, der Druck auf 0,5 bar gesenkt und sofort 50 ml der gemäss Beispiel 1 hergestellten Katalysatorlösung unter absoluter $O_2$-Abwesenheit eingesogen. Nach dem Aufdrücken von 40 bar Wasserstoff wurde unter Rühren und bei konstantem Druck (40 bar) während 1 Stunde bei 35 °C und 4 Stunden bei 45 °C hydriert. Nach total 5 Stunden Hydrierzeit wurde die hellgelbe Hydrierlösung aus dem Autoklaven gedrückt und der Autoklav anschliessend 3 mal mit je 100 ml Toluol nachgespült. Die vereinigten Tolyollösungen wurden am Rotationsverdampfer bei 60 °C/17 mbar eingedampft. Der Rückstand (210 g) wurde bei 130°-150 °C Badtemperatur und 12 mbar flachdestilliert. Man erhielt 201,6 g (99,3 %) R-(α-Hydroxy-β,β-dimethyl-γ-butyrolacton (= R-(—)-Pantolacton) mit einer optischen Reinheit von 90,7 %. $[\alpha]_D^{20}$ = —46,8° (c = 3 in $H_2O$).

Beispiel 3

In zu Beispiel 1 analoger Weise wurde eine Katalysatorlösung (100 ml) hergestellt aus 29,0 mg (0,0625 mMol) Chloronorbornadien-rhodium(I)-dimer, 70,0 mg (0,126 mMol) (2S,4S)-4-(Diphenylphosphino)-2-[(diphenylphosphino)methyl]-1-(tert. butoxycarbonyl)-pyrrolidin (BPPM) und 27,8 mg (0,126 mMol) Silbertrifluoracetat.

Beispiel 4

In einer Glove-Box wurde ein 500 ml-Autoklav mit 40 g (0,31 Mol) Ketopantalacton, 200 ml Toluol und 10 ml Katalysatorlösung (gemäss Beispiel 3) beladen. Die Hydrierung wurde bei 30 °C, einem konstanten Druck von 40 bar $H_2$ und unter intensivem Rühren durchgeführt. Die Temperatur der Lösung stieg bis auf 43 °C. Nach 1 Stunde betrug der Umsatz 98 %. Nach total 3 Stunden wurde analog Beispiel 2 aufgearbeitet, wobei man reines (R)-Pantolacton erhielt. $[\alpha]_D^{20}$ = —46,0°. Optische Reinheit 89,1 %.

Beispiel 5

In zu Beispiel 1 analoger Weise wurde eine Katalysatorlösung (100 ml) hergestellt aus 23,8 mg (0,0625 mMol) Bis-(acetonitril)-1,5-cyclooctadiene-rhodiumtetrafluoroborat, 75,3 mg (0,0625 mMol) (2S,4S)-4-(Di-m-Tolylphosphino)-2-[(di-m-tolylphosphino)methyl]-1-(diphenylphosphinoyl)-pyrrolidin ($mCH_3$-POPPM) und 22,2 mg (0,0625 mMol) Tetrabutylammoniumtrifluoracetat.

Beispiel 6

Die Hydrierung von 40,0 g Ketopantolacton mit 10 ml nach Beispiel 5 hergestellter Katalysatorlösung wurde wie in Beispiel 4 beschrieben durchgeführt. Nach einer Hydrierzeit von 2 Stunden wurde analog Beispiel 2 aufgearbeitet, wobei man reines (R)-Pantolacton erhielt. $[\alpha]_D^{20}$ = — 46,7°. Optische Reinheit 90,5 %.

Beispiel 7

In analoger Weise zu Beispiel 1 wurde eine Katalysatorlösung (100 ml) hergestellt aus 29,0 mg (0,0625 mMol) Bis(1,5-Cyclooctadien)rhodium-hexafluorophosphat, 45,3 mg (0,0625 mMol) $mCH_3$-POPPM und 25,3 mg (0,0625 mMol) Tetrabutylammoniumperfluorpropionat.

Beispiel 8

Die Hydrierung von 40 g Ketopantolacton mit 10 ml gemäss Beispiel 7 hergestellter Katalysatorlösung wurde wie in Beispiel 4 beschrieben durchgeführt. Nach einer Reaktionszeit von 3 Stunden wurde analog Beispiel 2 aufgearbeitet. Man erhielt reines (R)-Pantolacton. $[\alpha]_D^{20}$ = — 48,3°. Optische Reinheit 93,6 %.

Beispiel 9

In analoger Weise zu Beispiel 1 wurde eine Katalysatorlösung (100 ml) hergestellt aus 29,0 mg (0,0625 mMol) Bis(1,5-Cyclooctadien)rhodium hexafluorophosphat, 45,3 mg (0,0625 mMol) $mCH_3$-POPPM und 28,5 mg (0,0625 mMol) Tetrabutylammoniumperfluorbutyrat.

Beispiel 10

Die Hydrierung von 40,0 g Ketopantolacton mit 10 ml nach Beispiel 9 hergestellter Katalysatorlösung wurde wie in Beispiel 4 beschrieben durchgeführt. Nach einer Hydrierzeit von 3 Stunden wurde analog Beispiel 2 aufgearbeitet, wobei man reines (R)-Pantolacton erhielt. $[\alpha]_D^{20}$ = — 48,4°. Optische Reinheit 93,8 %.

Beispiel 11

In analoger Weise zu Beispiel 1 wurde eine Katalysatorlösung (100 ml) hergestellt aus 29,0 mg (0,0625 mMol) $[Rh(COD)_2]PF_6$, 45,3 mg (0,0625 mMol) $mCH_3$-POPPM und 23,0 mg (0,0625 mMol) Tetrabutylammoniumchlordifluoracetat.

Beispiel 12

Die Hydrierung von 40,0 g Ketopantolacton mit 10 ml nach Beispiel 11 hergestellter Katalysatorlösung wurde wie in Beispiel 4 beschrieben durchgeführt. Nach einer Hydrierzeit von 2 Stunden wurde analog Beispiel 2 aufgearbeitet, wobei man reines (R)-Pantolacton erhielt. $[\alpha]_D^{20}$ = — 46,9°. Optische Reinheit 91,0 %.

Beispiel 13

In zu Beispiel 1 analoger Weise wurde eine Katalysatorlösung (100 ml) hergestellt aus 58,0 mg (0,125 mMol) $[Rh(COD)_2]PF_6$, 90 mg (0,125 mMol) $mCH_3$-POPPM und 44,0 mg (0,125 mMol) Tetrabutylammoniumtrifluoracetat.

Beispiel 14

Die Hydrierung von 40 g Ketopantolacton mit 10 ml nach Beispeil 13 hergestellter Katalysatorlösung wurde analog Beispeil 4, jedoch bei einem Druck von 5 bar $H_2$ durchgeführt. Nach einer Hydrierzeit von 10 Stunden wurde analog Beispeil 2 aufgearbeitet. Man erhielt reines (R)-Pantolacton. $[\alpha]_D^{20}$ = — 47,7°. Optische Reinheit 92,2 %.

Beispiel 15

In zu Beispiel 13 analoger Weise wurde eine Lösung des Katalysators in Essigester hergestellt.

## Beispiel 16

Die Hydrierung von 40 g Ketopantolacton mit 10 ml der nach Beispiel 15 hergestellten Katalysatorlösung bei 30 °C/40 bar $H_2$ während 20 Stunden und Aufarbeitung gemäss Beispiel 2 ergab 97,5 %iges (R)-Pantolacton. $[\alpha]_D^{20}$ = — 46,8°. Optische Reinheit 90,7 %.

## Beispiel 17

In einer Glove-Box ($O_2$-Gehalt < 1 ppm) wurde ein 500 ml Stahlautoklav beladen mit 40,0 g (0,31 Mol) Ketopantolacton, 210 ml Toluol, 180,6 mg (0,389 mMol) $[Rh(COD)_2]PF_6$, 237,3 mg (0,389 mMol) m$CH_3$-BPPM und 138,3 mg (0,389 mMol) Tetrabutylammoniumtrifluoracetat. Die Hydrierung wurde bei einem konstanten Druck von 40 bar $H_2$, bei 30 °C und unter intensivem Rühren durchgeführt. Durch die Exothermie der Reaktion stieg die Temperatur bis auf 55 °C. Ein Umsatz von über 99 % war nach 1,2 Stunden erreicht. Nach einer Hydrierzeit von total 2 Stunden wurde wie in Beispiel 2 beschrieben aufgearbeitet. Man erhielt reines (R)-Pantolacton. $[\alpha]_D^{20}$ = — 46,0°. Optische Reinheit 89,1 %.

## Beispiel 18

In einer Glove-Box ($O_2$-Gehalt < 1 ppm) wurde ein 500 ml-Stahlautoklav beladen mit 40,0 g (0,31 Mol) Ketopantolacton, 210 ml Toluol, 180,6 mg (0,389 mMol) $[Rh(COD)_2]PF_6$, 254,3 mg (0,389 mMol) POPPM und 138,3 mg (0,389 mMol) Tetrabutylammoniumtrifluoracetat. Die Hydrierung wurde bei einem konstanten Druck von 40 bar $H_2$, bei 30 °C und unter intensivem Rühren durchgeführt. Durch die Exothermie der Reaktion stieg die Temperatur bis auf 58 °C. Ein Umsatz von über 99 % war nach 1 Stunde erreicht. Nach einer Hydrierzeit von total 2 Stunden wurde wie in Beispiel 2 beschrieben aufgearbeitet. Man erhielt reines (R)-Pantolacton. $[\alpha]_D^{20}$ = — 46,6°. Optische Reinheit 90,4 %.

## Beispiel 19

In zu Beispiel 18 analoger Weise wurde die Hydrierung unter Verwendung von 180,6 mg (0,389 mMol) $[Rh(COD)_2]PF_6$, 299,4 mg (0,389 mMol) (2S, 4S)-4-(Diphenylphosphino)-2-[(diphenylphosphino)methyl]-1-(di-p-carbomethoxyphenylphosphinoyl)-pyrrolidin anstelle von POPPM durchgeführt. Nach einer Reaktionszeit von 2 Stunden wurde analog Beispiel 2 aufgearbeitet, wobei man reines (R)-Pantolacton erhielt. $[\alpha]_D^{20}$ = — 46,2°. Optische Reinheit 89,6 %.

## Beispiel 20

In zu Beispiel 18 analoger Weise wurde die Hydrierung unter Verwendung von 336,2 mg (0,389 mMol) $[Rh(COD)(BPPM)]ClO_4$ und 138,3 mg (0,389 mMol) Tetrabutylammoniumtrifluoracetat durchgeführt. Nach einer Reaktionszeit von 2 Stunden wurde analog Beispiel 2 aufgearbeitet, wobei man reines (R)-Pantolacton erhielt. $[\alpha]_D^{20}$ = — 47,5°. Optische Reinheit 92,0 %.

## Beispiel 21

In zu Beispiel 18 analoger Weise wurde die Hydrierung von 40 g Ketopantolacton unter Verwendung von 241,1 mg (0,389 mMol) (2S, 4S)-4-(Diphenylphosphino)-2-[(diphenylphosphino)methyl]-1-(10-undecenoyl)-pyrrolidin, 144,7 mg (0,389 mMol) Tetrabutylammoniumchlordifluoracetat und 180,6 mg (0,389 mMol) $[Rh(COD)_2]PF_6$ durchgeführt. Nach einer Reaktionszeit von 1,5 Stunden wurde analog Beispiel 2 aufgearbeitet, wobei man reines (R)-Pantolacton erhielt. $[\alpha]_D^{20}$ = — 46,0°. Optische Reinheit 89,2 %.

## Beispiel 22

In einer Glove-Box ($O_2$-Gehalt < 1 ppm) wurde ein 500 ml-Stahlautoklav beladen mit 2,6 g (20 mMol) Itaconsäure, 150 ml Toluol, 50 ml Methanol, 2,02 g (20 mMol) Triäthylamin, 36,0 mg (0,0886 mMol) $[Rh(COD)_2]BF_4$, 49 mg (0,0886 mMol) BPPM und 31,5 mg (0,0886 mMol) Tetrabutylammoniumtrifluoracetat. Die Hydrierung wurde bei einem konstanten Druck von 6 bar $H_2$, bei 30 °C und unter intensivem Rühren durchgeführt. Nach einer Hydrierzeit von total 3 Stunden wurde die Reaktionslösung eingedampft und der Rückstand in 40 ml 2N Natronlauge gelöst. Die wässerige Phase wurde mit Toluol gewaschen, mit konz. Salzsäure auf pH 2 gestellt, mit NaCl gesättigt und mit 3 × 100 ml Aether extrahiert. Die vereinigten Aetherphasen wurden über $Na_2SO_4$ getrocknet und eingedampft. Man erhielt 2,4 g (92,3 %) Methylbernsteinsäure als weisse Kristalle. Smp. 111°-112 °C. $[\alpha]_D^{20}$ = — 16,2° (c = 5, EtOH). Optische Reinheit 95,4 %.

Beispiel 23

In einer Glove-Box wurde ein Autoklav beladen mit 5,87 g (20 mMol) trans-α-Acetamido-4-acetoxy-3-methoxy-zimtsäure, 200 ml Aethanol, 60,7 mg (0,6 mMol) Triäthylamin, 81,2 mg (0,20 mMol) $[Rh(COD)_2]BF_4$, 121,8 mg (0,22 mMol) BPPM und 71,1 mg (0,20 mMol) Tetrabutylammoniumtrifluoracetat. Die Hydrierung wurde bei 30 °C und 50 bar $H_2$ während 3 Stunden durchgeführt. Die Reaktionslösung wurde eingedampft und der Rückstand (5,6 g) in 40 ml 4,5 %iger wässriger $NaHCO_3$-Lösung gelöst. Die wässrige Phase wurde mit Toluol gewaschen, mit konz. HCl auf pH 3 gestellt, mit NaCl gesättigt und mit Chloroform extrahiert. Die organische Phase wurde über $Na_2SO_4$ getrocknet und eingedampft. Man erhielt 4,85 g (83 %) 3-(4-Acetoxy-3-methoxyphenyl)-N-acetyl-D-alanin als hellgelbe Kristalle. Smp. 174°-175 °C. $[\alpha]_D^{20}$ = — 36,7° (c = 1, $CH_3OH$). Optische Reinheit 90,6 %.

Beispiel 24

In einer Glove-Box ($O_2$-Gehalt < 1 ppm) wurde ein 500 ml-Stahlautoklav beladen mit 40,0 g (0,31 Mol) Ketopantolacton, 205 ml Tetrahydrofuran, 180,6 mg (0,389 mMol) $[Rh(COD)_2]PF_6$, 215,2 mg (0,389 mMol) BPPM und 138,3 mg Tetrabutylammoniumtrifluoracetat. Die Hydrierung erfolgte analog zu Beispiel 18. Nach einer Reaktionszeit von 1 Stunde wurde analog zu Beispiel 2 aufgearbeitet, wobei man reines (R)-Pantolacton erhielt. $[\alpha]_D^{20}$ = — 47,8°. Optische Reinheit 92,7 %.

Beispiel 25

In zu Beispiel 1 analoger Weise wurde eine Katalysatorlösung (100 ml) hergestellt aus 29 mg (0,0625 mMol) $[Rh(COD)_2]PF_6$, 45,3 mg (0,0625 mMol) $mCH_3$POPPM und 41,4 mg (0,0625 mMol) Tetrabutylammoniumperfluorcaprylat.

Beispiel 26

Die Hydrierung von 40,0 g (0,31 Mol) Ketopantolacton mit 10 ml nach Beispiel 25 hergestellter Katalysatorlösung wurde wie in Beispiel 4 beschrieben durchgeführt. Nach einer Hydrierzeit von 3 Stunden wurde analog Beispiel 2 aufgearbeitet, wobei man reines (R)-Pantolacton erhielt. $[\alpha]_D^{20}$ = — 48,3°. Optische Reinheit 93,6 %.

Beispiel 27

In einer Glove-Box ($O_2$-Gehalt < 1 ppm) wurde ein 500 ml-Stahlautoklav beladen mit 40,0 g (0,31 Mol) Ketopantolacton, 210 ml Toluol, 180,6 mg (0,389 mMol) $[Rh(COD)_2]PF_6$, 215,2 mg (0,389 mMol) BPPM und 176,4 mg (0,389 mMol) Tetrabutylammoniumpentafluorbenzoat. Die Hydrierung wurde bei einem konstanten Druck von 40 bar $H_2$, bei 30 °C und unter intensivem Rühren durchgeführt. Durch die Exothermie der Reaktion stieg die Temperatur bis auf 40 °C. Nach einer Hydrierzeit von 3 Stunden wurde wie in Beispiel 2 beschrieben aufgearbeitet. Man erhielt reines (R)-Pantolacton. $[\alpha]_D^{20}$ = — 45,8°. Optische Reinheit 88,8 %.

Beispiel 28

In einer Glove-Box ($O_2$-Gehalt < 1 ppm) wurden 40,1 mg (0,062 mMol) einer 40 %igen wässerigen Tetrabutylammoniumhydroxid-Lösung, 22,0 mg (0,062 mMol) Perfluorheptansäure, 25,1 mg (0,062 mMol) Bis(1,5-Cyclooctadien)rhodium(I)tetrafluoroborat und 44,7 mg (0,062 mMol) m-$CH_3$POPPM in einen 100 ml Messkolben gegeben. Dann wurde mit ca. 100 ml $O_2$-freiem Toluol auf die 100 ml-Marke aufgefüllt. Die erhaltene Suspension wurde anschliessent während 24 Stunden bei 22 °C gerührt, wobei sich eine orange, nahezu klare Lösung bildete.

Beispiel 29

In einer Glove-Box wurde ein 500 ml-Autoklav mit 40 g (0,31 Mol) Ketopantolacton, 200 ml Toluol und 10 ml nach Beispiel 28 hergestellter Katalysatorlösung beladen. Die Hydrierung wurde bei 40 °C, einem konstanten Druck von 40 bar $H_2$ und unter intensivem Rühren durchgeführt. Nach 1 Stunde betrug der Umsatz 99,9 %. Nach total 2,5 Stunden wurde analog Beispiel 2 aufgearbeitet, wobei man reines (R)-Pantolacton erhielt. $[\alpha]_D^{20}$ = — 47,5°. Optische Reinheit 92,0 %.

Beispiel 30

In zu Beispiel 28 analoger Weise wurde eine Katalysatorlösung (100 ml) hergestellt aus 40,1 mg (0,062 mMol) einer 40 %iger wässerigen Tetrabutylammoniumhydroxid-Lösung, 29,0 mg (0,062 mMol) Perfluornonansäure, 25,1 mg (0,062 mMol) Bis(1,5-Cyclooctadien)rhodium(I)tetrafluoroborat und 44,7 mg (0,062 mMol) m-$CH_3$POPPM.

Beispiel 31

Die Hydrierung von 40,0 g Ketopantolacton mit 10 ml nach Beispiel 30 hergestellter Katalysatorlösung wurde wie in Beispiel 29 beschrieben durchgeführt. Nach einer Hydrierzeit von 2,5 Stunden wurde analog Beispiel 2 aufgearbeitet, wobei man reines (R)-Pantolacton erhielt. $[\alpha]_D^{20}$ = — 47,5°. Optische Reinheit 92,0 %.

Beispiel 32

In zu Beispiel 28 analoger Weise wurde eine Katalysatorlösung (100 ml) hergestellt aus 40,1 mg (0,062 mMol) einer 40 %igen wässerigen Tetrabutylammoniumhydroxid-Lösung, 31,8 mg (0,062 mMol) Perfluordecansäure, 25,1 mg (0,062 mMol) Bis(1,5-Cyclooctadien)rhodium(I)tetrafluoroborat und 44,7 mg (0,062 mMol) m-$CH_3$POPPM.

Beispiel 33

Die Hydrierung von 40,0 g Ketopantolacton mit 10 ml nach Beispiel 32 hergestellter Katalysatorlösung wurde wie in Beispiel 29 beschrieben durchgeführt. Nach einer Hydrierzeit von 2,5 Stunden wurde analog Beispiel 2 aufgearbeitet, wobei man reines (R)-Pantolacton erhielt. $[\alpha]_D^{20}$ = — 47,8°. Optische Reinheit 92,7 %.

Beispiel 34

In zu Beispiel 28 analoger Weise wurde eine Katalysatorlösung (50 ml) hergestellt aus 127,1 mg (0,195 mMol) einer 40 %igen wässerigen Tetrabutylammoniumhydroxid-Lösung, 32,3 mg (0,195 mMol) α-Fluorzimtsäure, 78,5 mg (0,195 mMol) Bis(1,5-Cyclooctadien)rhodium(I)tetrafluoroborat und 140,9 mg (0,195 mMol) m-$CH_3$POPPM.

Beispiel 35

Die Hydrierung von 20,0 g Ketopantolacton, gelöst in 55 ml Toluol, mit 50 ml nach Beispiel 34 hergestellter Katalysatorlösung wurde wie in Beispiel 29 beschrieben durchgeführt. Nach einer Hydrierzeit von einer Stunde wurde analog Beispiel 2 aufgearbeitet, wobei man reines (R)-Pantolacton erhielt. $[\alpha]_D^{20}$ = — 46,4°. Optische Reinheit 89,9 %.

Beispiel 36

In zu Beispiel 28 analoger Weise wurde eine Katalysatorlösung (100 ml) hergestellt aus 254,2 mg (0,389 mMol) einer 40 %igen wässerigen Tetrabutylammoniumhydroxid-Lösung, 65,4 mg (0,389 mMol) 2-Fluor-3-phenylpropansäure, 157,8 mg (0,389 mMol) Bis(1,5-Cyclooctadien)rhodium(I)tetrafluoroborat und 281,0 mg (0,389 mMol) m-$CH_3$POPPM.

Beispiel 37

Die Hydrierung von 40,0 g Ketopantolacton, gelöst in 110 ml Toluol, mit 100 ml nach Beispiel 36 hergestellter Katalysatorlösung wurde wie in Beispiel 29 beschrieben durchgeführt. Nach einer Hydrierzeit von 2 Stunden wurde analog Beispiel 2 aufgearbeitet, wobei man reines (R)-Pantolacton erhielt. $[\alpha]_D^{20}$ = — 47,9°. Optische Reinheit 92,8 %.

Beispiel 38

In einer Glove-Box ($O_2$-Gehalt < 1 ppm) wurde ein 500 ml-Stahlautoklav beladen mit 10,2 mg (0,1 Mol) Brenztraubensäuremethylester, 200 ml Benzol, 32,1 mg (0,0496 mMol) Di-μ-trifluoroacetat -bis-(1,5-cyclooctadien)di-rhodium und 56,0 mg (0,0991 mMol) BPPM. Die Hydrierung wurde bei einem konstanten Druck von 20 bar $H_2$, bei 21 °C und unter intensivem Rühren durchgeführt. Der Umsatz nach 4 Stunden betrug 98,4 %. Die Reaktionslösung wurde eingedampft und der Rückstand bei Atmosphärendruck destilliert. Man erhielt (R)-(+)-Milchsäuremethylester (Sdp. 143-145°). Zur Bestimmung des Enantiomerenüberschusses im Gaschromatogramm, wurde eine Probe davon quantitativ in den Camphansäureester übergeführt. Enantiomerenüberschuss : 80,1 %.

Beispiel 39

Als Vergleich wurde die Hydrierung von 10,2 g Brenztraubensäuremethylester in zu Beispiel 38 analoger Weise mit 24,5 mg (0,0496 mMol) [Rh(COD) Cl]$_2$ anstatt des Rhodiumtrifluoracetat-Komplexes durchgeführt. Man erhielt R-(+)-Milchsäuremethylester (Sdp. 143-145°). Enantiomerenüberschuss :

70,6 %. Für diese Hydrierung unter identischen Bedingungen wird in der Literatur eine optische Ausbeute von 66,3 % angegeben (I. Ojima, T. Kogure u. K. Achiwa, J. Chem. Soc., Chem. Commun., 1977, 428).

Beispiel 40

In zu Beispiel 38 analoger Weise wurde die Hydrierung von 11,6 g (0,1 Mol) Brenztraubensäureäthylester durchgeführt. Umsatz 99,2 % nach 23 Stunden. Man erhielt (R)-(+)-Milchsäureäthylester (Sdp. 152°-154°). Enantiomerenüberschuss : 79,5 %.

Beispiel 41

In zu Beispiel 39 analoger Weise wurde die Hydrierung von 11,6 g Brenztraubensäureäthylester durchgeführt. Man erhielt (R)-(+)-Milchsäureäthylester (Sdp. 151-154°). Enantiomerenüberschuss : 71,2 %. Für die Hydrierung unter identischen Bedingungen wird in der Literatur eine optische Ausbeute von 65,3 % angegeben (K. Achiwa, Tetrahedron Lett. 1977, 3735).

Beispiel 42

In einer Glove-Box ($O_2$-Gehalt < 1 ppm) wurden zu einer Lösung von 4,02 mg (0,0062 mMol) $[Rh(COD) CF_3COO]_2$ in 20 ml Toluol 57,6 mg eines Copolymeren (Phosphorgehalt 1,33 %), hergestellt aus (2S,4S)-4-(Diphenylphosphino)-2-[(diphenylphosphino)methyl]-1-(4-pentenoyl)-pyrrolidin und 2-Hydroxyäthylmethacrylat, gegeben und die erhaltene Suspension während 24 Stunden bei 22 °C gerührt.

Beispiel 43

Die Hydrierung von 20,0 g Ketopantolacton mit der nach Beispiel 42 hergestellten Katalysator-Suspension, wurde wie in Beispiel 29 beschrieben bei 60 °C durchgeführt. Nach einer Hydrierzeit von 24 Stunden wurde analog Beispiel 2 aufgearbeitet, wobei man reines (R)-Pantolacton erhielt. $[\alpha]_D^{20}$ = —43,6°. Optische Reinheit 84,4 %.

**Patentansprüche** (für die Vertragsstaaten : BE, CH, DE, FR, GB, IT, LI, NL)

1. Chirale Rhodium-diphosphinkomplexe der allgemeinen Formel

$$[Rh\ (X)\ (Y)\ (L_{0,\ 1\ oder\ 2})]_{1\ oder\ 2} \qquad (I)$$

worin X, welches gegebenenfalls auf einem Träger fixiert sein kann, einen Rest der Formel Z—COOdarstellt, worin Z wiederum die Gruppe

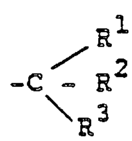

Perfluorphenyl, Perfluorbiphenyl oder einen Rest der Formel

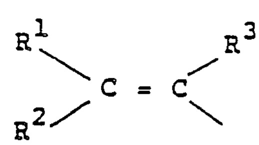

bedeutet und $R^1$, $R^2$ und $R^3$ Halogen, niederes Alkyl, Perfluorphenyl, Perfluor-$C_{1-20}$-Alkyl, Wasserstoff, oder die Gruppe —COA oder AOC—$(CF_2)_n$— darstellen, worin A die Reste —OR oder —NR'$_2$ bedeutet, wobei jedoch wenigstens einer der Substituenten $R^1$, $R^2$ und $R^3$ Fluor bedeutet, R Wasserstoff, niederes Alkyl oder ein Kation, R' Wasserstoff oder niederes Alkyl, und n eine Zahl 1 bis 20 bedeuten und worin weiter Y einen chiralen Diphosphinliganden und L einen neutralen Liganden darstellen, wobei die niederen Alkylreste 1-9 Kohlenstoffatome aufweisen.

2. Chirale Komplexe gemäss Anspruch 1, worin Z die Gruppe

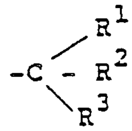

darstellt.

3. Chirale Komplexe gemäss Anspruch 1 oder 2, worin wenigstens zwei der Substituenten $R^1$, $R^2$ und $R^3$ Fluor darstellen.

4. Chirale Komplexe gemäss einem der Ansprüche 1-3, worin X in der Formel I die Gruppe $CF_3$—COO$^-$ oder Perfluor-$C_{1-20}$-Alkyl darstellt.

5. Chirale Komplexe gemäss einem der Ansprüche 1-4, worin Y in der Formel I einen chiralen Liganden der allgemeinen Formel

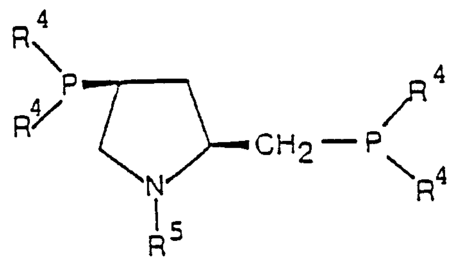

(II)

darstellt, worin $R^4$ Aryl und $R^5$ die Gruppen —CO—$R^6$, —$SO_2$—$R^6$, —$PO(R^6)_2$ oder —$PS(R^6)_2$ darstellen, wobei $R^6$ Aryl, niederes Alkyl, di-Arylamino, di-niederes Alkylamino, Aryloxy oder niederes Alkoxy bedeutet, wobei die niederen Alkyl- bzw. Alkoxyreste 1-9 Kohlenstoffatome aufweisen und der Ausdruck Aryl für gegebenenfalls Substituiertes Phenyl steht.

6. Chirale Komplexe gemäss Anspruch 5, worin $R^5$ die Gruppe —$PO(R^6)_2$ darstellt.

7. Chirale Komplexe gemäss Anspruch 5 oder 6, worin $R^4$ m-Tolyl und $R^6$ Phenyl bedeuten.

8. Verfahren zur Herstellung von chiralen Rhodium-diphosphinkomplexen der allgemeinen Formel

$$[Rh\ (X)\ (Y)\ (L_{0,1\ \text{oder}\ 2})]_{1\ \text{oder}\ 2} \qquad \text{(I)}$$

worin X, welches gegebenenfalls auf einem Träger fixiert sein kann, einen Rest der Formel Z—COO$^-$ darstellt, worin Z wiederum die Gruppe

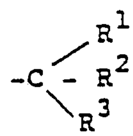

Perfluorphenyl, Perfluorbiphenyl oder einen Rest der Formel

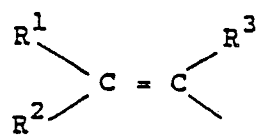

bedeutet und $R^1$, $R^2$ und $R^3$ Halogen, niederes Alkyl, Perfluorphenyl, Perfluor-$C_{1-20}$-Alkyl, Wasserstoff oder die Gruppe —COA oder AOC—$(CF_2)_n$— darstellen, worin A die Reste —OR oder —$NR'_2$ bedeutet, wobei jedoch wenigstens einer der Substituenten $R^1$, $R^2$ und $R^3$ Fluor bedeutet, R Wasserstoff, niederes Alkyl oder ein Kation, R' Wasserstoff oder niederes Alkyl, und n eine Zahl 1 bis 20 bedeuten und worin weiter Y einen chiralen Diphosphinliganden und L einen neutralen Liganden darstellen, wobei die niederen Alkylreste 1-9 Kohlenstoffatome aufweisen,

dadurch gekennzeichnet, dass man

a) einen Rhodiumkomplex der Formel

$$[Rh\ (X)\ (L_m)]_{1\ \text{oder}\ 2} \qquad \text{(III)}$$

worin X und L obige Bedeutung haben und m eine Zahl von 1 bis 4 bedeutet,
mit einem chiralen Diphosphinliganden umsetzt, oder

b) einen Rhodiumkomplex der Formel

$$[Rh\ (L_{m+1})]^+\ A^- \qquad \text{(IV)}$$

worin L und m obige Bedeutung haben und $A^-$ ein Anion, insbesondere $BF_4^-$, $ClO_4^-$, $PF_6^-$ oder $B(C_6H_5)_4^-$ darstellt,

mit einem chiralen Diphosphinliganden und einem das Anion X enthaltenden Salz umsetzt, oder

c) einen Rhodium-diphosphinkomplex der Formel

$$[Rh\ (L_p)\ (Y)]^+\ A^- \qquad (V)$$

worin L, Y und $A^-$ obige Bedeutung haben und p eine Zahl von 1 bis 3 darstellt, mit einem das Anion X enthaltenden Salz umsetzt, oder

d) einen chiralen Rhodium-diphosphinkomplex der Formel

$$[Rh\ (X^1)\ (L_{1\ oder\ 2})\ (Y)] \qquad (VI)$$

worin $X^1$ Halogen bedeutet und L und Y obige Bedeutung haben, mit einem Silbersalz oder Thalliumsalz der Formeln

$$Ag—X\ oder\ Tl—X \qquad (VII)$$

worin X obige Bedeutung hat, umsetzt.

9. Chirale Rhodium-diphosphinkomplexe der allgemeinen Formel I gemäss den Ansprüchen 1-7, erhältlich gemäss dem Verfahren nach Anspruch 8.

10. Verwendung der chiralen Rhodium-diphosphinkomplexe der Formel I gemäss den Ansprüchen 1-7, als Katalysatoren bei asymmetrischen Hydrierungen.

**Patentansprüche** (für den Vertragsstaat Oesterreich)

1. Verfahren zur Herstellung von chiralen Rhodium-diphosphinkomplexen der allgemeinen Formel

$$[Rh\ (X)\ (Y)\ (L_{0,1\ oder\ 2})]_{1\ oder\ 2} \qquad (I)$$

worin X, welches gegebenenfalls auf einem Träger fixiert sein kann, einen Rest der Formel Z—COO⁻ darstellt, worin Z wiederum die Gruppe

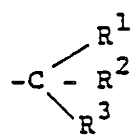

Perfluorphenyl, Perfluorbiphenyl oder einen Rest der Formel

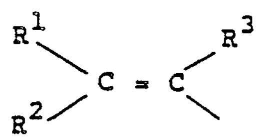

bedeutet und $R^1$, $R^2$ und $R^3$ Halogen, niederes Alkyl, Perfluorphenyl, Perfluor-$C_{1-20}$-Alkyl, Wasserstoff, oder die Gruppe —COA oder AOC—$(CF_2)_n$— darstellen, worin A die Reste —OR oder —$NR'_2$ bedeutet, wobei jedoch wenigstens einer der Substituenten $R^1$, $R^2$ und $R^3$ Fluor bedeutet, R Wasserstoff, niederes Alkyl oder ein Kation, R' Wasserstoff oder niederes Alkyl, und n eine Zahl 1 bis 20 bedeuten und worin weiter Y einen chiralen Diphosphinliganden und L einen neutralen Liganden darstellen, wobei die niederen Alkylreste 1-9 Kohlenstoffatome aufweisen,
dadurch gekennzeichnet, dass man

a) einen Rhodiumkomplex der Formel

$$[Rh\ (X)\ (L_m)]_{1\ oder\ 2} \qquad (III)$$

worin X und L obige Bedeutung haben und m eine Zahl von 1 bis 4 bedeutet, mit einem chiralen Diphosphinliganden umsetzt, oder

b) einen Rhodiumkomplex der Formel

$$[Rh\ (L_{m+1})]^+\ A^- \qquad (IV)$$

worin L und m obige Bedeutung haben und $A^-$ ein Anion, insbesondere $BF_4^-$, $ClO_4^-$, $PF_6^-$ oder $B(C_6H_5)_4^-$ darstellt,

mit einem chiralen Diphosphinliganden und einem das Anion X enthaltenden Salz umsetzt, oder
c) einen Rhodium-diphosphinkomplex der Formel

$$[Rh\ (L_p)\ (Y)]^+\ A^- \qquad (V)$$

worin L, Y und $A^-$ obige Bedeutung haben und p eine Zahl von 1 bis 3 darstellt,
mit einem das Anion X enthaltenden Salz umsetzt, oder
d) einen chiralen Rhodium-diphosphinkomplex der Formel

$$[Rh\ (X^1)\ (L_{1\ oder\ 2})\ (Y)] \qquad (VI)$$

worin $X^1$ Halogen bedeutet und L und Y obige Bedeutung haben,
mit einem Silbersalz oder Thalliumsalz der Formeln

$$Ag—X \text{ oder } Tl—X \qquad (VII)$$

worin X obige Bedeutung hat,
umsetzt.

2. Verfahren gemäss Anspruch 1, dadurch gekennzeichnet, dass man Ausgangsmaterialien verwendet, worin Z in dem Rest X die Gruppe

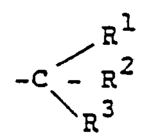

bedeutet.

3. Verfahren gemäss Anspruch 1 oder 2, dadurch gekennzeichnet, dass man Ausgangsmaterialien verwendet, worin wenigstens zwei der Substituenten $R^1$, $R^2$ und $R^3$ Fluor darstellen.

4. Verfahren gemäss einem der Ansprüche 1 bis 3, dadurch gekennzeichnet, dass man Ausgangsmaterialien verwendet, worin X die Gruppe $CF_3—COO^-$ oder Perfluor-$C_{1-20}$-Alkyl darstellt.

5. Verfahren gemäss einem der Ansprüche 1 bis 4, dadurch gekennzeichnet, dass der Diphosphinligand Y einen chiralen Liganden der allgemeinen Formel

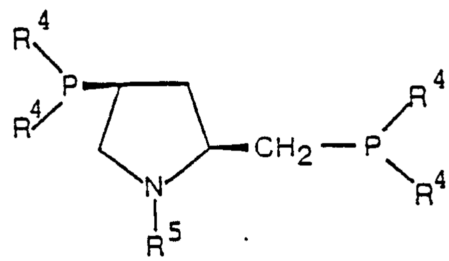

(II)

darstellt, worin $R^4$ Aryl und $R^5$ die Gruppen $—CO—R^6$, $—SO_2—R^6$, $—PO(R^6)_2$ oder $—PS(R^6)_2$ darstellen, wobei $R^6$ Aryl, niederes Alkyl, di-Arylamino, di-niederes Alkylamino, Aryloxy oder niederes Alkoxy bedeutet, wobei die niederen Alkyl- bzw. Alkoxyreste 1-9 Kohlenstoffatome aufweisen und der Ausdruck Aryl für gegebenenfalls substituiertes Phenyl steht.

6. Verfahren gemäss Anspruch 5, dadurch gekennzeichnet, dass in dem Liganden der Formel II $R^5$ die Gruppe $—PO(R^6)_2$ darstellt.

7. Verfahren gemäss Anspruch 5 oder 6, dadurch gekennzeichnet, dass in dem Liganden der Formel II $R^4$ m-Tolyl und $R^6$ Phenyl bedeuten.

8. Verwendung der gemäss den Ansprüchen 1-7 herstellbaren chiralen Rhodiumdiphosphinkomplexe der Formel I, als Katalysatoren bei asymmetrischen Hydrierungen.

9. Verwendung der gemäss den Ansprüchen 1-7 herstellbaren chiralen Rhodiumdiphosphinkomplexe der Formel I, bei der asymmetrischen Hydrierung von Ketopantolacton zu Pantolacton.

**Claims** (for the Contracting States : BE, CH, DE, FR, GB, IT, LI, NL)

1. Chiral rhodium-diphosphine complexes of the general formula

$$[Rh\ (X)\ (Y)\ (L_{0,1\ or\ 2})]_{1\ or\ 2} \qquad (I)$$

wherein X, which can be optionally fixed to a carrier, represents a residue of the formula Z—COO⁻, wherein Z signifies the group

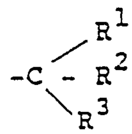

perfluorophenyl, perfluorobiphenyl or a residue of the formula

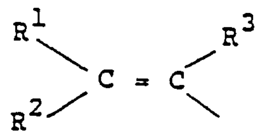

and $R^1$, $R^2$ and $R^3$ represent halogen, lower alkyl, perfluorophenyl, perfluoro-$C_{1-20}$-alkyl, hydrogen or the group —COA or AOC—$(CF_2)_n$— in which A signifies the residue —OR or —$NO'_2$, with the proviso that at least one of the substituents $R^1$, $R^2$ and $R^3$ signifies fluorine, R signifies hydrogen, lower alkyl or a cation, R′ signifies hydrogen or lower alkyl and n signifies a number 1 to 20 and wherein Y represents a chiral diphosphine ligand and L represents a neutral ligand, whereby the lower alkyl residues have 1-9 carbon atoms.

2. Chiral complexes in accordance with claim 1, wherein Z represents the group

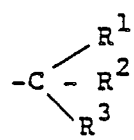

3. Chiral complexes in accordance with claim 1 or 2, wherein at least two of the substituents $R^1$, $R^2$ and $R^3$ represent fluorine.

4. Chiral complexes in accordance with any one of claims 1-3, wherein X in formula I represents the group $CF_3$—COO⁻ or perfluoro-$C_{1-20}$-alkyl.

5. Chiral complexes in accordance with any one of claims 1-4, wherein Y in formula I represents a chiral ligand of the general formula

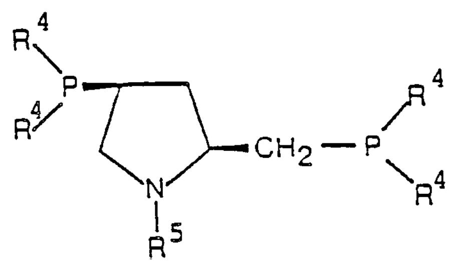

(II)

wherein $R^4$ represents aryl and $R^5$ represents the groups —CO—$R^6$, —$SO_2$—$R^6$, —$PO(R^6)_2$ or —$PS(R^6)_2$ in which $R^6$ signifies aryl, lower alkyl, di-arylamino, di-lower alkylamino, aryloxy or lower alkoxy, whereby the lower alkyl and alkoxy residues have 1-9 carbon atoms and the term aryl stands for optionally substituted phenyl.

6. Chiral complexes in accordance with claim 5, wherein $R^5$ represents the group —$PO(R^6)_2$.

7. Chiral complexes in accordance with claim 5 or 6, wherein $R^4$ signifies m-tolyl and $R^6$ signifies phenyl.

8. A process for the manufacture of chiral rhodium-diphosphine complexes of the general formula

$$[Rh\ (X)\ (Y)\ (L_{0,1\ or\ 2})]_{1\ or\ 2} \qquad (I)$$

wherein X, which can be optionally fixed to a carrier, represents a residue of the formula Z—COO⁻, wherein Z signifies the group

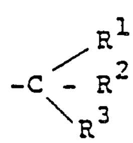

perfluorophenyl, perfluorobiphenyl or a residue of the formula

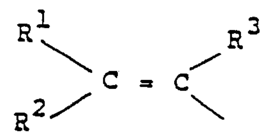

and $R^1$, $R^2$ and $R^3$ represent halogen, lower alkyl, perfluorophenyl, perfluoro-$C_{1-20}$-alkyl, hydrogen or the group —COA or AOC—$(CF_2)_n$— in which A signifies the residue —OR or —$NR'_2$, with the proviso that at least one of the substituents $R^1$, $R^2$ and $R^3$ signifies fluorine, R signifies hydrogen, lower alkyl or a cation, R′ signifies hydrogen or lower alkyl and n signifies a number 1 to 20 and wherein Y represents a chiral diphosphine ligand and L represents a neutral ligand, whereby the lower alkyl residues have 1-9 carbon atoms,
characterized by

a) reacting a rhodium complex of the formula

$$[Rh\ (X)\ (L_m)]_{1\ or\ 2} \qquad (III)$$

wherein X and L have the above significance and m signifies a number of 1 to 4, with a chiral diphosphine ligand, or

b) reacting a rhodium complex of the formula

$$[Rh\ (L_{m+1})]^+\ A^- \qquad (IV)$$

wherein L and m have the above significance and $A^-$ represents an anion, especially $BF_4^-$, $ClO_4^-$, $PF_6^-$ or $B(C_6H_5)_4^-$,
with a chiral diphosphine ligand and a salt containing the anion X, or

c) reacting a rhodium-diphosphine complex of the formula

$$[Rh\ (L_p)\ (Y)]^+\ A^- \qquad (V)$$

wherein L, Y and $A^-$ have the above significance and p represents a number of 1 to 3, with a salt containing the anion X, or

d) reacting a chiral rhodium-diphosphine complex of the formula

$$[Rh\ (X^1)\ (L_{1\ or\ 2})\ (Y)] \qquad (VI)$$

wherein $X^1$ signifies halogen and L and Y have the above significance,
with a silver salt or thallium salt of the formulae

$$Ag—X\ or\ Tl—X \qquad (VII)$$

wherein X has the above significance.

9. Chiral rhodium-diphosphine complexes of general formula I in accordance with claims 1-7, obtainable in accordance with the process according to claim 8.

10. The use of the chiral rhodium-diphosphine complexes of formula I in accordance with claims 1-7 as catalysts in asymmetric hydrogenations.

**Claims** (for the Contracting State AT)

1. A process for the manifacture of chiral rhodium-diphosphine complexes of the general formula

$$[Rh\ (X)\ (Y)\ (L_{0,1\ or\ 2})]_{1\ or\ 2} \qquad (I)$$

wherein X, which can be optionally fixed to a carrier, represents a residue of the formula Z—$COO^-$, wherein Z signifies the group

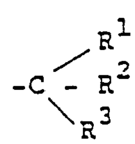

perfluorophenyl, perfluorobiphenyl or a residue of the formula

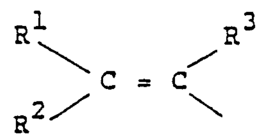

and $R^1$, $R^2$ and $R^3$ represent halogen, lower alkyl, perfluorophenyl, perfluoro-$C_{1-20}$-alkyl, hydrogen or the group —COA or AOC—$(CF_2)_n$— in which A signifies the residue —OR or —NO'$_2$, with the proviso that at least one of the substituents $R^1$, $R^2$ and $R^3$ signifies fluorine, R signifies hydrogen, lower alkyl or a cation, R' signifies hydrogen or lower alkyl and n signifies a number 1 to 20 and wherein Y represents a chiral diphosphine ligand and L represents a neutral ligand, whereby the lower alkyl residues have 1-9 carbon atoms, characterized by

a) reacting a rhodium complex of the formula

$$[Rh\ (X)\ (L_m)]_{1\ or\ 2} \qquad (III)$$

wherein X and L have the above significance and m signifies a number of 1 to 4, with a chiral diphosphine ligand, or

b) reacting a rhodium complex of the formula

$$[Rh\ (L_{m+1})]^+\ A^- \qquad (IV)$$

wherein L and m have the above significance and $A^-$ represents an anion, especially $BF_4^-$, $ClO_4^-$, $PF_6^-$ or $B(C_6H_5)_4^-$, with a chiral diphosphine ligand and a salt containing the anion X, or

c) reacting a rhodium-diphosphine complex of the formula

$$[Rh\ (L_p)\ (Y)]^+\ A^- \qquad (V)$$

wherein L, Y and $A^-$ have the above significance and p represents a number of 1 to 3, with a salt containing the anion X, or

d) reacting a chiral rhodium-diphosphine complex of the formula

$$[Rh\ (X^1)\ (L_{1\ or\ 2})\ (Y)] \qquad (VI)$$

wherein $X^1$ signifies halogen and L and Y have the above significance, with a silver salt or thallium salt of the formulae

$$Ag—X\ or\ Tl—X \qquad (VII)$$

wherein X has the above significance.

2. A process in accordance with claim 1, characterized in that starting materials in which Z in the residue X signifies the group

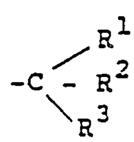

are used.

3. A process in accordance with claim 1 or 2, characterized in that starting materials in which at least two of the substituents $R^1$, $R^2$ and $R^3$ represent fluorine are used.

4. A process in accordance with any one of claims 1 to 3, characterized in that starting materials in which X represents the group $CF_3$—COO$^-$ or perfluoro-$C_{1-20}$-alkyl are used.

5. A process in accordance with any one of claims 1 to 4, characterized in that the diphosphine ligand Y represents a chiral ligand of the general formula

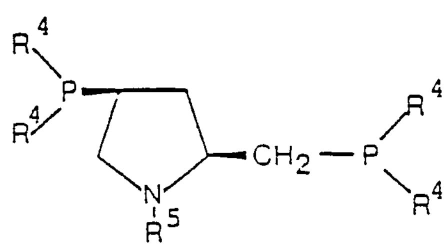

(II)

wherein $R^4$ represents aryl and $R^5$ represents the groups $—CO—R^6$, $—SO_2—R^6$, $—PO(R^6)_2$ or $—PS(R^6)_2$ in which $R^6$ signifies aryl, lower alkyl, di-arylamino, di-lower alkylamino, aryloxy or lower alkoxy, whereby the lower alkyl and alkoxy residues have 1-9 carbon atoms and the term aryl stands for optionally substituted phenyl.

6. A process in accordance with claim 5, characterized in that in the ligands of formula II $R^5$ represents the group $—PO(R^6)_2$.

7. A process in accordance with claim 5 to 6, characterized in that in the ligands of formula II $R^4$ signifies m-tolyl and $R^6$ signifies phenyl.

8. The use of the chiral rhodium diphosphine complexes of formula I manufacturable in accordance with claims 1-7 as catalysts in asymmetric hydrogenations.

9. The use of the chiral rhodium diphosphine complexes of formula I manufacturable in accordance with claims 1-7 in the asymmetric hydrogenation of ketopantolactone to pantolactone.

**Revendications** (pour les États contractants : BE, CH, DE, FR, GB, IT, LI, NL)

1. Complexes chiraux de rhodium-diphosphines de formule générale

$$[Rh\ (X)\ (Y)\ (L_{0,1\ ou\ 2})]_{1\ ou\ 2} \qquad (I)$$

dans laquelle X, qui peut le cas échéant être fixé sur un support, représente un radical de formule Z—COO⁻ dans lequel Z représente lui-même le groupe

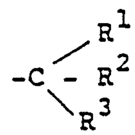

un groupe perfluorophényle, perfluorobiphényle ou un radical de formule

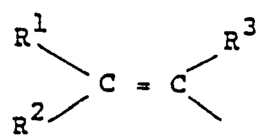

et $R^1$, $R^2$ et $R^3$ représentent des halogènes, des groupes alkyle inférieurs, perfluorophényle, perfluoralkyle en C1-C20, l'hydrogène ou le groupe —COA ou $AOC—(CF_2)_n—$ dans lequel A représente les groupes —OR ou $—NR'_2$, l'un au moins des symboles $R^1$, $R^2$ et $R^3$ représentant le fluor, R représente l'hydrogène, un groupe alkyle inférieur ou un cation, R' représente l'hydrogène ou un groupe alkyle inférieur et n est un nombre allant de 1 à 20, Y représente un ligand chiral consistant en une diphosphine et L représente un ligand neutre, les groupes alkyle inférieurs contenant 1 à 9 atomes de carbone.

2. Complexes chiraux selon la revendication 1, dans lesquels Z représente le groupe

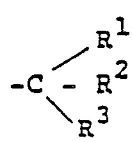

3. Complexes chiraux selon la revendication 1 ou 2, dans lesquels au moins deux des symboles $R^1$, $R^2$ et $R^3$ représente le fluor.

4. Complexes chiraux selon l'une des revendications 1 à 3, dans lesquels X représente, dans la formule I, le groupe $CF_3—COO^-$ ou un groupe perfluoralkyle en C1-C20.

5. Complexes chiraux selon l'une des revendications 1 à 4, dans lesquels le symbole Y de la formule I représente un ligand chiral de formule générale

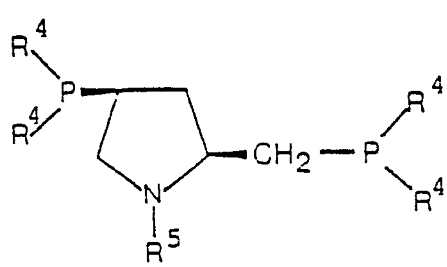

(II)

dans laquelle $R^4$ représente un groupe aryle et $R^5$ les groupes —CO—$R^6$, —$SO_2$—$R^6$, —$PO(R^6)_2$ ou —$PS(R^6)_2$, $R^6$ représentant un groupe aryle, alkyle inférieur, di-arylamino, di-(alkyle inférieur)-amino, aryloxy ou alcoxy inférieur, les groupes alkyle et alcoxy inférieurs contenant 1 à 9 atomes de carbone et les groupes aryle consistant en groupes phényle éventuellement substitués.

6. Complexes chiraux selon la revendication 5, dans lesquels $R^5$ représente le groupe —$PO(R^6)_2$.

7. Complexes chiraux selon la revendication 5 ou 6, dans lesquels $R^4$ représente un groupe m-tolyle et $R^6$ un groupe phényle.

8. Procédé de préparation de complexes chiraux rhodium-diphosphines de formule générale

$$[Rh\ (X)\ (Y)\ (L_{0,1\ ou\ 2})]_{1\ ou\ 2} \qquad (I)$$

dans laquelle X, qui peut le cas échéant être fixé sur un support, représente un radical de formule Z—COO— dans lequel Z représente lui-même le groupe

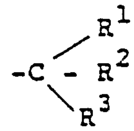

un groupe perfluorophényle, perfluorobiphényle ou un groupe de formule

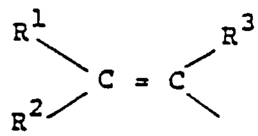

et $R^1$, $R^2$ et $R^3$ représentent des halogènes, des groupes alkyle inférieurs, perfluorophényle, perfluoralkyle en C1-C20, l'hydrogène ou le groupe —COA ou $AOC(CF_2)_n$— dans lequel A représente les groupes —OR ou —$NR'_2$, l'un au moins des symboles $R^1$, $R^2$ et $R^3$ représentant le fluor, R représente l'hydrogène, un groupe alkyle inférieur ou un cation, R' représente l'hydrogène ou un groupe alkyle inférieur et n est un nombre allant de 1 à 20, Y représente un ligand chiral consistant en une diphosphine et L représente un ligand neutre, les groupes alkyle inférieurs contenant 1 à 9 atomes de carbone, caractérisé en ce que

a) on fait réagir un complexe de rhodium de formule

$$[Rh\ (X)\ (L_m)]_{1\ ou\ 2} \qquad (III)$$

dans laquelle X et L ont les significations indiquées ci-dessus et m est un nombre allant de 1 à 4, avec un ligand chiral consistant en une diphosphine, ou bien

b) on fait réagir un complexe de rhodium de formule

$$[Rh\ (L_{m+1})]^+\ A^- \qquad (IV)$$

dans laquelle L et m ont des significations indiquées ci-dessus et $A^-$ représente un anion, en particulier $BF_4^-$, $ClO_4^-$, $PF_6^-$ ou $B(C_6H_5)_4^-$, avec un ligand chiral consistant en une diphosphine et un sel contenant l'anion X, ou bien

c) on fait réagir un complexe rhodium-diphosphine de formule

$$[Rh\ (L_p)\ (Y)]^+\ A^- \qquad (V)$$

dans laquelle L, Y et $A^-$ ont les significations indiquées ci-dessus et p est un nombre allant de 1 à 3, avec un sel contenant l'anion X, ou bien

d) on fait réagir un complexe chiral rhodium-diphosphine de formule

$$[Rh\ (X^1)\ (L_{1\ ou\ 2})\ (Y)] \qquad (VI)$$

dans laquelle $X^1$ représente un halogène et L et Y ont les significations indiquées ci-dessus, avec un sel d'argent ou un sel de thallium de formule respective

$$Ag—X\ ou\ Tl—X \qquad (VII)$$

dans lesquelles X a les significations indiquées ci-dessus.

9. Complexes chiraux rhodium-diphosphines de formule générale I selon les revendications 1 à 7, obtenables par le procédé selon la revendication 8.

10. Utilisation des complexes chiraux rhodium-diphosphines de formule I selon les revendications 1 à 7 en tant que catalyseurs dans des hydrogénations asymétriques.

**Revendications** (pour l'Etat contractant AT)

1. Procédé de préparation de complexes chiraux rhodium-diphosphines de formule générale

$$[Rh\ (X)\ (Y)\ (L_{0,1\ ou\ 2})]_{1\ ou\ 2} \qquad (I)$$

dans laquelle X, qui peut le cas échéant être fixé sur un support, représente un radical de formule Z—COO⁻ dans lequel Z représente lui-même le groupe

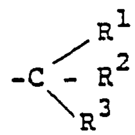

un groupe perfluorophényle, perfluorobiphényle ou un groupe de formule

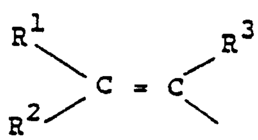

et $R^1$, $R^2$ et $R^3$ représentent des halogènes, des groupes alkyle inférieurs, perfluorphényle, perfluoralkyle en C1-C20, l'hydrogène ou le groupe —COA ou AOC—$(CF_2)_n$— dans lequel A représente les groupes —OR ou —$NR'_2$, l'un au moins des symboles $R^1$, $R^2$ et $R^3$ représentant le fluor, R représente l'hydrogène, un groupe alkyle inférieur ou un cation, R' représente l'hydrogène ou un groupe alkyle inférieur et n est un nombre allant de 1 à 20, Y représente un ligand chiral consistant en une diphosphine et L un ligand neutre, les groupes alkyle inférieurs contenant 1 à 9 atomes de carbone,
caractérisé en ce que

a) on fait réagir un complexe de rhodium de formule

$$[Rh\ (X)\ (L_m)]_{1\ ou\ 2} \qquad (III)$$

dans laquelle X et L ont les significations indiquées ci-dessus et m est un nombre allant de 1 à 4, avec un ligand chiral consistant en une diphosphine, ou bien

b) on fait réagir un complexe de rhodium de formule

$$[Rh\ (L_{m+1})]^+\ A^- \qquad (IV)$$

dans laquelle L et m ont les significations indiquées ci-dessus et $A^-$ représente un anion, en particulier $BF_4^-$, $ClO_4^-$, $PF_6^-$ ou $B(C_6H_5)_4^-$,
avec un ligand chiral consistant en une diphosphine et un sel contenant l'anion, ou bien

c) on fait réagir un complexe rhodium-diphosphine de formule

$$[Rh\ (L_p)\ (Y)]^+\ A^- \qquad (V)$$

dans laquelle L, Y et $A^-$ ont les significations indiquées ci-dessus et p est un nombre allant de 1 à 3, avec un sel contenant l'anion X, ou bien

d) on fait réagir un complexe chiral rhodium-diphosphine de formule

$$[Rh\ (X^1)\ (L_{1\ ou\ 2})\ (Y)] \qquad (VI)$$

dans laquelle $X^1$ représente un halogène et L et Y ont les significations indiquées ci-dessus, avec un sel d'argent ou un sel de thallium de formule respective

$$Ag—X\ ou\ Tl—X \qquad (VII)$$

dans lesquelles X a les significations indiquées ci-dessus.

2. Procédé selon la revendication 1, caractérisé en ce que l'on utilise des produits de départ dans lesquels Z représente, dans le groupe X, le groupe

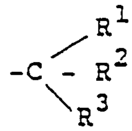

3. Procédé selon la revendication 1 ou 2, caractérisé en ce que l'on utilise des produits de départ dans lesquels au moins deux des symboles $R^1$, $R^2$ et $R^3$ représentent le fluor.

4. Procédé selon l'une des revendications 1 à 3, caractérisé en ce que l'on utilise des produits de départ dans lesquels X représente le groupe $CF_3$—COO— ou un groupe perfluoralkyle en C1-C20.

5. Procédé selon les revendications 1 à 4, caractérisé en ce que le ligand Y consistant en une diphosphine est un ligand chiral de formule générale

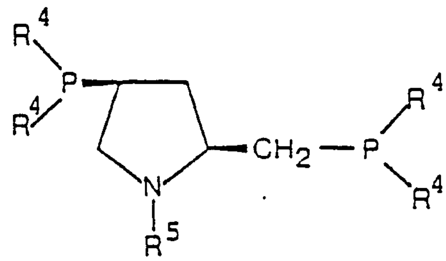

(II)

dans laquelle $R^4$ représente un groupe aryle et $R^5$ représente les groupes —CO—$R^6$, —$SO_2$—$R^6$, —$PO(R^6)_2$ ou —$PS(R^6)_2$, $R^6$ représente un groupe aryle, alkyle inférieur, diarylamino, di-(alkyle inférieur)-amino, aryloxy ou alcoxy inférieur, les groupes alkyle et alcoxy inférieur contenant 1 à 9 atomes de carbone et les groupes aryle consistant en groupes phényle éventuellement substitués.

6. Procédé selon la revendication 5, caractérisé en ce que, dans le ligand de formule II, $R^5$ représente le groupe —$PO(R^6)_2$.

7. Procédé selon la revendication 5 ou 6, caractérisé en ce que, dans le ligand de formule II, $R^4$ représente un groupe m-tolyle et $R^6$ un groupe phényle.

8. Utilisation des complexes chiraux rhodium-diphosphines de formule I, préparés selon les revendications 1 à 7, en tant que catalyseurs pour des hydrogénations asymétriques.

9. Utilisation des complexes chiraux rhodium-diphosphines de formule I préparés selon les revendications 1 à 7 pour l'hydrogénation asymétrique de la cétopantolactone en la pantolactone.